# EUROPÄISCHE PATENTANMELDUNG

(11) **EP 2 090 293 A1**
(43) Veröffentlichungstag der Anmeldung: **19.08.2009**
(21) Anmeldenummer: 09405003.6
(22) Anmeldetag: 12.01.2009
(51) Int. Cl.: A61K 8/73, A61Q 19/00, A61Q 19/06, A61Q 19/08, A61K 8/02

(54) **Wasserdispergierbarer oder wasserlöslicher Polymerfilm als Träger von dermatologischen und kosmetischen Wirkstoffen**

(30) Priorität: 07.02.2008 CH 1642008
(71) Anmelder: Mibelle AG, 5033 Buchs (CH)
(72) Erfinder: Irrgang, Bernhard, 5737 Menziken (CH); MacQuarrie, Reginald, Toronto, Ontario, M8Y2H2 (CA)
(74) Vertreter: Rottmann, Maximilian

(57) **Zusammenfassung**

Es werden mit mindestens einem dermatologischen oder kosmetischen Wirkstoff beladene wasserdispergierbare oder wasserlöslichere Polymerfilme und ein Verfahren zu deren Herstellung beschrieben. Als Wirkstoffe kommen insbesondere Anti-Cellulite-Wirkstoffe, feuchtigkeitsspendende Stoffe, Anti-Falten-Wirkstoffe und Stoffe zur Hautglättung in Frage.

## Beschreibung

Die Erfindung betrifft einen wasserdispergierbaren oder wasserlöslichen Polymerfilm, der als Träger von dermatologischen und kosmetischen Wirkstoffen dient. Anwendungsgebiete sind Anti-Cellulite-Behandlungen, Feuchtigkeitsanreicherungen für die Haut sowie Hautstraffung und Hautglättung, z.B. zur Reduktion von Falten.

Kosmetische Produkte gibt es in einer Vielzahl von Darreichungsformen. Kosmetische Produkte mit erhöhter Wirkung sind zumeist Emulsionen, die auf die Haut aufgetragen werden und dort auch verbleiben ("Leave-on"-Produkte). Dort werden auch die markantesten Wirkungen angetroffen. Bei Produkten, die nicht auf der Haut verbleiben ("Rinse-off"-Produkte), sind aufgrund der begrenzten Kontaktzeit hautkosmetische Formulierungen mit nur weniger starken Wirkungen möglich.

Im Allgemeinen sind "Rinse-off"-Produkte mit guten Wirkungen nicht anzutreffen. "Leave-on"-Produkte zeigen oft gute Wirkungen, weisen aber andere Nachteile auf.

So beschreibt die WO 2006/009987 A2 eine lösliche Filmzusammensetzung zur Anwendung auf der Haut, es werden ein System, ein Kit und ein Verfahren offenbart, mit dem eine wirksame Menge eines in Wasser instabilen Mittels auf die Haut aufgebracht werden kann.

Aus der US 2003/0224053 A1 sind Zusammensetzungen und Verfahren zur Freisetzung aktiver Mittel auf der Haut bekannt. Zu diesem Zweck wird eine Film bereitgestellt, der auf mindestens zwei Polyvinylalkoholen mit unterschiedlichen Viskositäten beruht. Durch die Verwendung der Polyvinylalkoholmischung kann auf ein weiteres Polymer verzichtet werden, um einen dünnen durchsichtigen Film zu schaffen.

Die US 2007/0154527 A1 betrifft selbsttragende, sich rasch auflösende Filme und Verfahren zur ihrer Herstellung, wobei die Filme ein topisches Mittel enthalten, wodurch der Film gleichmäßig verteilt werden kann. Die in dem Filme enthaltenen aktivenMittel können durch die Beschaffenheit des Films nicht in den Blutkreislauf gelangen und damit nur örtlich wirken.

In der US 2004/0228892 A1 werden kosmetische und Hautpflegeprodukte offenbart, die in einer Polymermatrix dispergiert sind oder von dieser eingeschlossen werden. Die Polymermatrix muß allerdings vor der Verwendung gelöst oder geschmolzen werden, um das kosmetische oder Hautpflegeprodukt freizusetzen. Des weiteren ist aufgrund des niedrigen Schmelzpunktes der hier verwendeten Polymere eine Verwendung im Sommer nicht möglich.

Die auf der Haut verbleibenden Emulsions-Bestandteile können durchaus zu Irritationen oder aufgrund der Okklusivität z.B. zur Verklebung von Hautporen führen.

Daher ergab sich als Aufgabe der vorliegenden Erfindung, "Rinse-off"-Produkte mit erhöhtem Wirkungsspektrum schaffen.

Diese Aufgabe wird erfindungsgemäss durch einen wasserdispergierbaren oder wasserlöslichen Polymerfilm, der als Träger von dermatologischen und kosmetischen Wirkstoffen dient, gelöst.

Aus der Veröffentlichung WO 2004/087857 sind zwar wasserlösliche Polymerfilme bekannt. Diese dienen jedoch dem Aufbringen von oberflächenaktiven Substanzen, insbesondere Seifen und Detergentien.

Das erfindungsgemässe Produkt ist ein dünner wasserdispergierbarer oder wasserlöslicher Polymerfilm, der sich bei Kontakt mit Wasser eng an die Haut anlegt. Im Allgemeinen löst sich der Polymerfilm bei weiterer Wasserzugabe komplett auf. Die Grösse und Dicke des Polymerfilmes hängt vom Anwendungszweck ab.

Bevorzugt ist auch ein Polymermultilayerfilm, der mindestens zwei wasserdispergierbare oder wasserlösliche Polymerfilme mit jeweils verschiedenen kosmetischen oder dermatologischen Wirkstoffen enthält. Dieser Multilayerfilm wird nach dem Aufbringen auf die Haut durch den Kontakt mit Wasser aufgrund des unterschiedlichen Lösungsverhaltens der jeweiligen Polymerfilme nacheinander aufgelöst und die entsprechenden Wirkstoffe werden nacheinander freigesetzt.

Der erfindungsgemässe wasserlösliche oder wasserdispergierbare mit mindestens einem kosmetischen oder dermatologischen Wirkstoff beladene Polymerfilm besteht aus:
(A) einem Polymer auf Pflanzenbasis als Grundkomponente und
(B) 1 bis 40 Gew.% eines kosmetischen oder dermatologischen Wirkstoffes, der in Form einer Emulsion, einem Gel oder einer Lösung in die Grundkomponente eingearbeitet ist.

Nachfolgend werden die einzelnen Komponenten des Polymerfilms beschrieben.

Die Grundkomponente, also die Filmpolymer-Basis ("Film polymer base"), umfaßt viele unterschiedliche Polymere, die als Basis zur Herstellung von schnell auflösenden Filmen geeignet sind. Erfahrungsgemäss sind natürlich vorkommende Polymere und Hydrokolloide auf Basis von Kohlenhydraten und/oder Proteinen am besten geeignet. Dazu zählen z.B. Hydroxylpropylmethyl-Cellulosen, Natriumcarboxymethyl-Cellulosen, Carboxymethyl-Cellulosen, pflanzliche Proteine, wie Soyaproteine, Gelatine ("High bloom to low bloom") oder Milchproteine. Weiter zu nennen sind Alginate wie auch Hydroxylmethyl-Cellulose oder Hydroxylpropyl-Cellulose. Die Filmbasis kann aber auch aus Stärken verschiedenen Molgewichtes oder Carrageenanen, verschiedenen Galactomannanen ("Locust bean gum"), auf mikrobiellem Wege hergestellten Gums, wie Xanthan und Gellan gum, ebenso wie anderen unterschiedlichen Polymeren pflanzlicher Herkunft, wie Guar oder dessen Derivate, bestehen, je nach gewünschter Ausrichtung des Polymerfilmes und je nach gewünschten Auflöseeigenschaften mit polaren Flüssigkeiten, wie Wasser.

Andere mögliche Polymere umfassen die Gruppe der Polyacrylsäuren, Polyethylenoxide und Polyvinylalkohole und deren Derivate, wie Ether oder Ester.

Die Polymefilmbasis kann auch durch die Zugabe von Polyolen, die als Weichmacher dienen, in ihren Eigenschaften modifiziert werden, ebenso durch Einsatz von Emulgatoren und anderer Hilfsmittel. Damit sind Charakteristik, Eigenschaften und Produzierbarkeit des Polymerfilmes beeinflussbar.

Zubereitung von Präparaten mit Wirkstoffen

Der kosmetische oder dermatologische Wirkstoff wird in Form einer Emulsion, eines Gels oder einer Lösung in das Grundpolymer eingearbeitet.

Zur vorbeugenden und aktiven Behandlung von Hautunebenheiten, wie z.B. Cellulite, und zur Straffung der Haut sind eine Vielzahl von Wirkstoffen und Wirkstoffkombinationen bekannt. Alle bekannten Produkte dieser Art können in die oben beschriebenen Polymerfilme inkorporiert werden. Diese Stoffe sollen z.B. die Entwicklung von Cellulite verhindern oder verlangsamen. Dazu werden Stoffwechselvorgänge in den Zellen angeregt, wie auch die Blutzirkulation. Ebenso dienen diese Substanzen dazu, die Hautspannung und die Hautbeschaffenheit in einem guten Zustand zu erhalten oder zu verbessern.

Zu nennen sind Wirkstoffe wie z.B. Koffein, Carnitin, Extrakte und Auszüge aus Algen, z.B. *Spirulina platensis*, Pflanzen- und Fruchtextrakte, z.B. aus Mönchspfeffer, Mäusedom, Birke, Rosskastanie, Tigergras, Efeu, Schachtelhalm, um nur eine kleine Auswahl zu nennen. Daneben können auch einzelne oder Kombinationen von Vitaminen, wie Folsäure, Vitamin E, Vitamine der B-Reihe, Vitamin C, Ubiquinon (Q 10) sowie Mineralien, wie z.B. Calcium, Chrom, Magnesium, Kalium, Mangan, Molybdän, Selen, Jod, Zink, Mangan, eingesetzt werden. Zu nennen sind auch Substanzen, welche zur Hautglättung unterstützend wirken können, wie z.B. Hyaluronsäure und Vitamin-A-Derivate.

Diese Wirkstoffe werden idealerweise in die Grundrezeptur des Polymerfilms eingearbeitet, die entweder eine Emulsion, ein Gel oder eine Lösung sein kann.

Des weiteren kann enthält der Polymerfilm
(C) 1 - 30 Gew.-% Tenside und/oder Benetzungsmittel,
(D) 0.01 - 20 Gew.-% Modifizierungsmittel
(E) 1 - 30 Gew.-% feuchtigkeitsspendende Mittel oder Öle und/oder
(I) antimikrobielle Stoffe, Perfums, Farbstoffe.

### Tenside und Benetzungsmittel

Es können eine Vielzahl von Stoffen aus dieser Klasse Verwendung finden. Zu nennen sind anionische, nichtionische, amphotere Tenside und Kombination aus Stoffen dieser Gruppen. Gewöhnlich wird der Einsatz von weit verbreiteten Tensiden, wie Natriumlaurylsulfaten oder Natriumlaurylethersulfaten, bevorzugt. Die gewünschte Funktionalität des Produktes kann aber den Einsatz anderer oder weiterer Tenside erfordern. Ebenso ist eine weitere Kombination mit in der Kosmetik gebräuchlichen Emulgatoren und Estern möglich, beispielsweise mit sekundären Alkanesulfonaten, Cocoylgluconaten, Lauroylglutamaten, Methyltauriden, Natriumcocoylisethionaten, Tributylethersulfaten, C₁₂-C₁₆-Alkoholethoxylaten, Iso-C₁₀-alkoholethoxylat, C₁₁-Alkoholethoxylat oder Cocamidopropylbetain.

### Modifizierungsmittel

Hier sind zu nennen: Farbstoffe, Mittel, welche für ein besseres Hautgefühl eingesetzt werden können, wie kationische Polymere, Silicone und natürliche Öle, sowie Parfums und Mittel zur Konservierung gemäss Anhang 6 der EU-Kosmetikverordnung. Weitere Hilfsstoffe zur Modifizierung des Polymerfilmes können Antioxidantien und Vitamine, z.B. die Vitamine A, E oder C, sowie UV-Filter gemäss Anhang 2 der EU-Kosmetikverordnung sein.

### Feuchtigkeitsspendende Mittel und Öle

Hier sind zu nennen: Emolliensester, Seidenproteinderivate und verschiede Phosphatester, ebenso *Aloe vera*-Extrakte, natürliche Pflanzenöle, Sojaölderivate. Verwendet werden können auch verschiedene Fettsäuren und deren Derivate, Kakaobutter, Sheabutter, Kokosnuss- und Palmkernöle, Canolaöle und weitere natürliche Öle.

Diese Bestandteile können direkt zum Brei (Slurry), der zur Herstellung des Polymerfilmes verwendet wird, zugegeben werden.

Im Folgenden wird ein typisches Herstellungsverfahren beschrieben.
1. Die Basis des Polymerfilmes wird je nach Art des Polymers entweder zu heissem oder kaltem Wasser zugegeben, um einen glatten, homogenen Brei (Slurry) zu bilden.
2. Im nächsten Schritt werden je nach Bedarf und Anwendung zusätzliche Komponenten, wie Tenside, Öle, Emulgatoren, Konservierungsmittel, Feuchtigkeitsmittel, Plastifizierungsmittel, Farbstoffe oder Parfums, zugefügt.
3. Unter weiterem Rühren werden im letzten Schritt die gewünschten Wirkstoffe zur Zubereitung zugegeben.
4. Der Ansatz wird weiter gerührt bis eine vollständig homogene Masse oder Flüssigkeit erhalten wird. Je nach Anforderungsprofil kann noch ein zusätzlicher Entgasungsschritt angeschlossen werden, um eventuell eingeschlossene Luft zu entfernen.
5. Die Masse wird anschliessend auf ein Heizband gleichmässig verteilt und bis auf den gewünschten Flüssigkeitsgehalt getrocknet. Typischerweise weist ein Polymerfilm einen Feuchtigkeitsgehalt von 5 bis 15 %, idealerweise von 3 bis 10 %, auf.
6. Die Masse kann, abhängig von den Anforderungen der Produktionseinrichtungen und des Produktes, auf verschiedene Weise auf das Heizband aufgebracht werden, z.B. mittels mechanischer Verstreichvorrichtungen, durch Extrusion oder Aufwalzen.
7. Unterstützend können zur Ausformung des Polymerfilmes auch Gase, wie Luft, Stickstoff oder andere Gase, eingesetzt werden.
8. Die Polymerfilme können je nach Anforderungen noch auf andere angewärmte Oberflächen, wie dünne Metallbänder, Bänder aus Kunststoff (PP/PET), Bänder aus beschichtetem Papier (Mylar), aufgebracht und dort verfestigt werden. Auf diese Weise können auch Oberflächenstrukturen und Anfärbungen eingearbeitet werden.
9. Die so entstandenen Polymerfilme können zur Verhinderung des Zusammenklebens mit dafür geeigneten Substanzen, wie z.B. pflanzlicher Stärke oder Talk (Talkumpuder), bestäubt werden.
10. Das Fertigprodukt kann zur Endverarbeitung noch geschnitten und/oder bedruckt werden.

Die auf diese Weise hergestellten Polymerfilme können zu Tuben, Säckchen und anderen Formen weiterverarbeitet werden. Dazu können die Polymerfilme mittels Hitze verschweisst werden.

### Die Polymerfilme weisen folgende physikalische Parameter auf:

Die Dicke der Polymerfilme ist je nach Produktanforderungen variabel. Normalerweise liegt die Dicke zwischen 15 und 600 Mikrometern.

Die Sprödigkeit bzw. Bruchfestigkeit der Polymerfilme kann variiert werden. Typische Bruchkräfte liegen zwischen 2 und 50 psi. (13790 bis 344738 Pa).

Elastizität: Durch Einsatz von Weichmachern und Anpassung der eingesetzten Basisformulierungen bestehen Variationsmöglichkeiten.

### Beispiele

### Beispiel 1

**Anti-Cellulite-Film - Typ 1**

| | | | | |
|---|---|---|---|---|
| Wasser | 15 | bis | 30 | Gew.-% |
| Carboxymethylcellulose | 7 | bis | 25 | Gew.-% |
| Alginate | 25 | bis | 45 | Gew.-% |
| Zubereitung mit Wirkstoffen | 5 | bis | 15 | Gew.-% |
| Tensid | 2,5 | bis | 10 | Gew.-% |
| Emolliens | 20 | bis | 35 | Gew.-% |
| Duftstoffe | 1 | bis | 8 | Gew.-% |
| Farbstoffe | 0,01 | bis | 0,20 | Gew.-% |

### Beispiel 2

**Anti-Falten Film - Typ 2**

| | | | | |
|---|---|---|---|---|
| Wasser | 10 | bis | 25 | Gew.-% |
| Carboxymethylcellulose | 15 | bis | 35 | Gew.-% |
| Alginate | 5 | bis | 20 | Gew.-% |
| Zubereitung mit Wirkstoffen | 10 | bis | 30 | Gew.-% |
| Tensid | 7 | bis | 20 | Gew.-% |
| Emolliens | 20 | bis | 35 | Gew.-% |
| Duftstoffe | 2 | bis | 10 | Gew.-% |
| Farbstoffe | 0,01 | bis | 0,10 | Gew.-% |

### Beispiel 3

**Anti-Cellulite-Film - Typ 3**

| | | | | |
|---|---|---|---|---|
| Wasser | 13 | bis | 30 | Gew.-% |
| Carboxymethylcellulose | 5 | bis | 20 | Gew.-% |
| Alginate | 30 | bis | 40 | Gew.-% |
| Zubereitung mit Wirkstoffen | 20 | bis | 35 | Gew.-% |
| Tensid | 5 | bis | 15 | Gew.-% |
| Emolliens | 20 | bis | 30 | Gew.-% |
| Duftstoffe | 1 | bis | 7 | Gew.-% |
| Farbstoffe | 0,02 | bis | 0,10 | Gew.-% |

### Beispiel 4

**Anti-Cellulite-Film - Typ 4**

| | | | | |
|---|---|---|---|---|
| Wasser | 15 | bis | 30 | Gew.-% |
| Carboxymethylcellulose | 10 | bis | 25 | Gew.-% |
| Alginate | 10 | bis | 25 | Gew.-% |
| Zubereitung mit Wirkstoffen | 25 | bis | 35 | Gew.-% |
| Tensid | 5 | bis | 20 | Gew.-% |
| Emolliens | 5 | bis | 15 | Gew.-% |
| Duftstoffe | 3 | bis | 10 | Gew.-% |
| Farbstoffe | 0,05 | bis | 0,10 | Gew.-% |

### Beispiel 5

**Anti-Falten-Film - Typ 5**

| | | | | |
|---|---|---|---|---|
| Wasser | 10 | bis | 25 | Gew.-% |
| Carboxymethylcellulose | 25 | bis | 30 | Gew.-% |
| Alginate | 5 | bis | 15 | Gew.-% |
| Zubereitung mit Wirkstoffen | 10 | bis | 25 | Gew.-% |
| Tensid | 5 | bis | 10 | Gew.-% |
| Emolliens | 15 | bis | 35 | Gew.-% |
| Duftstoffe | 2 | bis | 5 | Gew.-% |
| Farbstoffe | 0,01 | bis | 0,30 | Gew.-% |

### Beispiel 6

**Typische Zubereitung mit Wirkstoffen**

| | | | | |
|---|---|---|---|---|
| Wasser | 70 | bis | 90 | Gew.-% |
| Ethanol | 0 | bis | 10 | Gew.-% |
| Feuchtigkeitsspendende Agentien, | | | | |
| z.B. Glycerin | | | | |
| oder Sorbitol | 1 | bis | 15 | Gew.-% |
| Anti-Cellulite-Wirkstoff 1 | | | | |
| z.B. Koffein | 0,1 | bis | 5 | Gew.-% |
| Anti-Cellulite-Wirkstoff 2 | | | | |
| z.B. Amara Shape | 0,1 | bis | 5 | Gew.-% |
| Gelbildner | | | | |
| z.B. Polyquaternium-37 | 0,1 | bis | 3 | Gew.-% |
| Lösungsvermittler / Emulgator | | | | |
| z.B. Polyglycerylester | | | | |
| oder Sorbitanester | | | | |
| oder Fettalkoholethoxylate | 0,1 | bis | 5 | Gew.-% |
| Aromastoffe | | | | |
| z.B. Menthol | | | | |
| oder Pfefferminzöl | | | | |
| oder Orangenöl | | | | |
| oder andere Fruchtöle | 5 | bis | | Gew.-% |
| Konservierungsmittel | 0 | bis | 0,8 | Gew.-% |

### Beispiel 7

**Wirkstoffemulsion**

| | | | | |
|---|---|---|---|---|
| Wasser | 60 | bis | 90 | Gew.-% |
| Ethanol | 0 | bis | 10 | Gew.-% |
| Feuchtigkeitsspendende Agentien | | | | |
| z.B. Glycerin | | | | |
| oder Sorbitol | 1 | bis | 15 | Gew.-% |
| Anfl-Faltenwirkstoff 1 | | | | |
| z.B. Vitamin-A-Palmitate | 0,001 | bis | 3 | Gew.-% |
| Anü-Faltenwirkstoff 2 | | | | |
| z.B. Q10 | | | | |
| oder Sesamprotein | | | | |
| oder Fruchtextrakte | 0,0001 | bis | 3 | Gew.-% |
| pflanzliches Öl | | | | |
| z.B.: Palmöl | | | | |
| oder Olivenöl | | | | |
| oder Sheabutter | | | | |
| oder Sesamöl | 0,1 | bis | 15 | Gew.-% |
| Lösungsvermittler / Emulgator | | | | |
| z.B. Polyglycerylester | | | | |
| oder Sorbitanester | | | | |
| oder Benzyl/Benzoatester | | | | |
| oder Fettalkoholethoxylate | | | | |
| oder Zuckerderivate | 0,1 | bis | 15 | Gew.-% |
| Konsistenzgeber | | | | |
| z.B. Fettalkohole | | | | |
| oder Glyceride | | | | |
| oder Phosphate | | | | |
| oder Stärkederivate | | | | |
| oder Cellulosederivate | | | | |
| oder Guarderivate | 0 | bis | 5 | Gew.-% |
| Aromastoffe | | | | |
| z.B. Menthol | | | | |
| oder Pfefferminzöl | | | | |
| oder Orangenöl | | | | |
| oder andere Fruchtöle | 0,01 | bis | 5 | Gew.-% |
| UV-Filter | 0 | bis | 10 | Gew.-% |
| Konservierungsmittel | 0 | bis | 0,8 | Gew.-% |

**Beispiel 8 Anti-Cellulite-Film - Typ Mulitlayer**

| **Basisfilm** | | | | |
|---|---|---|---|---|
| Wasser | 15 | bis | 30 | Gew.-% |
| Carboxymethylcellulose | 10 | bis | 25 | Gew.-% |
| Alginate | 10 | bis | 25 | Gew.-% |
| Emolliens | 5 | bis | 15 | Gew.-% |
| Duftstoffe | 3 | bis | 10 | Gew.-% |
| Farbstoffe | 0,05 | bis | 0,10 | Gew.-% |

| **Toplayer** | | | | |
|---|---|---|---|---|
| Wasser | 15 | bis | 30 | Gew.-% |
| Carboxymethylcellulose | 10 | bis | 25 | Gew.-% |
| Alginate | 10 | bis | 25 | Gew.-% |
| Wirkstoffkonzentrat | 1 | bis | 15 | Gew.-% |
| Tensid | 5 | bis | 20 | Gew.-% |
| Emolliens | 5 | bis | 15 | Gew.-% |
| Duftstoffe | 3 | bis | 10 | Gew.-% |
| Farbstoffe | 0,05 | bis | 0,10 | Gew.-% |

| Bevorzugte Wirkstoffe sind: | |
|---|---|
| Produkt | Hersteller |
| Amara Shape | Mibelle Biochemistry |
| Iso-Slim-Komplex | Mibelle Biochemistry |
| Sesaflash | Seppic |
| Perfelline | Rahn |
| Liftonin | Rahn |
| Produkte der Cell-Activ-Reihe | Rahn |
| Astraforce | Mibelle Biochemistry |
| Perfection Peptide P3 | Mibelle Biochemistry |

## Patentansprüche

1. Wasserlöslicher oder wasserdispergierbarer mit mindestens einem kosmetischen oder dermatologischen Wirkstoff beladener Polymerfilm bestehend aus:
(A) einem Polymer auf Pflanzenbasis als Grundkomponente und
(B) 1 bis 40 Gew.% eines kosmetischen oder dermatologischen Wirkstoffes, der in Form einer Emulsion, einem Gel oder einer Lösung in die Grundkomponente eingearbeitet ist.

2. Polymerfilm nach Anspruch 1, bei dem die Grundkomponente ausgewählt wird aus der Gruppe bestehend aus Hydrokolloiden auf Basis von Kohlenhydraten und/oder Proteinen, pflanzlichen Proteinen, Stärken, Gelantine, Alginate, Carrageenanen, Galactomannanen (Locust bean gum), auf mikrobiellem Weg hergestellte Gums, pflanzlichen Polymeren.

3. Polymerfilm nach Anspruch 1 oder 2, bei dem die Grundkomponente ausgewählt wird aus der Gruppe bestehend aus Hydroxypropylmethyl-Cellulosen, Natriumcarboxymethyl-Cellulosen, Carboxymethyl-Cellulosen, Sojaproteinen, Milchproteinen, Hydroxylmethyl-Cellulosen, Hydroxylpropyl-Cellulosen, Xanthan, Gellan, Guar und dessen Derivate.

4. Polymerfilm nach Anspruch 1, bei dem der dermatologische oder kosmetische Wirkstoff ein Anti-Cellulite-Wirkstoff und/oder ein feuchtigkeitsspendender Stoff und/oder ein Anti-Falten-Wirkstoff und/oder ein Stoff zur Hautglättung ist.

5. Polymerfilm nach einem der Anspruch 1, bei dem der dermatologische oder kosmetische Wirkstoff ein Extrakt aus Pflanzen und/oder Algen und/oder Vitaminen und/oder deren Derivaten und/oder Hyaluronsäure und/oder ihrer Derivate und/oder Mineralien ist.

6. Polymerfilm nach einem der Ansprüche 1 bis 5, wobei der Polymerfilm einen Feuchtigkeitsgehalt von 5 bis 15%, vorzugsweise 3 bis 10% aufweist.

7. Polymerfilm nach einem der Ansprüche 1 bis 6, **dadurch gekennzeichnet, dass** er sowohl in kalten als auch in warmen Wasser schnelllöslich ist.

8. Polymerfilm nach einem der Ansprüche 1 bis 7, des weiteren enthaltend
(C) 1,0 - 30 Gew.-% Tenside und/oder Benetzungsmittel,
(D) 0,01 - 20 Gew.-% Modifizierungsmittel
(E) 1,0 - 30 Gew.-% feuchtigkeitsspendende Mittel oder Öle und/oder
(I) antimikrobielle Stoffe, Parfums, Farbstoffe.

9. Verwendung des wasserdispergierbaren oder wasserlöslichen Polymerfilms nach einem der Ansprüche 1 bis 8 als Träger, der mindestens einen dermatologischen oder kosmetischen Wirkstoff enthält, zur Hautglättung, zur Anregung von Stoffwechselvorgängen in den Hautzellen, zur Anregung Blutzirkulation, zur Behandlung von Cellulite oder zur Straffung der Haut.

10. Verwendung des wasserdispergierbaren oder wasserlöslichen Polymerfilms nach einem der Ansprüche 1 bis 8 in Form eines Polymermultilayerfilms, der mindestens zwei wasserdispergierbare oder wasserlösliche Polymerfilme mit jeweils verschiedenen kosmetischen oder dermatologischen Wirkstoffen enthält.

11. Verfahren zur Herstellung eines wasserlöslichen oder wasserdispergierbaren mit mindestens einem kosmetischen oder dermatolgischen Wirkstoff beladener Polymerfilm, umfassend die folgenden Verfahrensschritte:
i. das Grundpolymer wird, je nach Art des Polymers, in heißes oder kaltes Wasser gegeben, um einen glatten, homogenen Brei (Slurry) zu erhalten;
ii. zur diesem Brei werden, je nach Anwendung, weitere Komponenten, ausgewählt aus der Gruppe bestehend aus Tensiden, Ölen, Emulgatoren, Konservierungsmitteln, Feuchtigkeitsmitteln, Plastifizierungsmitteln, Farbstoffen oder Parfums, gemischt;
iii. der erhaltenen Zubereitung werden die dermatologisch oder kosmetisch aktiven Komponenten in Form einer Emulsion, einem Gel oder einer Lösung beigemischt;
iv. die erhaltene Mischung wird gerührt, bis eine vollständig homogene Masse oder Flüssigkeit erhalten wird;
v. zur Entfernung von eventuell eingeschlossener Luft wird ein zusätzlicher Entgasungsschritt durchgeführt; und anschließend
vi. die erhaltene Masse gleichmässig auf ein Heizband aufgebracht und bis auf den gewünschten Flüssigkeitsgehalt getrocknet wird.

12. Verfahren nach Anspruch 11, **dadurch gekennzeichnet, dass** der Polymerfilm auf einen Feuchtigkeitsgehalt von 5 bis 15 %, vorzugsweise 3 bis 10% getrocknet wird.

13. Verfahren nach einem der Ansprüche 11 oder 12, **dadurch gekennzeichnet, dass** das Aufbringen der Masse auf das Heizband mittels einer mechanischen Verstreichvorrichtung, durch Extrusion oder durch Aufwalzen erfolgt.

14. Verfahren nach einem der Ansprüche 11 bis 13, **dadurch gekennzeichnet, dass** die Ausformung des Polymerfilmes durch Gase, wie Luft, Stickstoff oder andere Inertgase, unterstützt wird.

15. Verfahren nach einem der Ansprüche 11 bis 14, **dadurch gekennzeichnet, dass** die Polymerfilme nach der Entfernung vom Heizband noch auf andere angewärmte Oberflächen, wie dünne Metallbänder, Bänder aus PP/PET oder Bänder aus mit Mylar beschichtetem Papier, aufgebracht und dabei verfestigt werden.

16. Verfahren nach Anspruch 15, **dadurch gekennzeichnet, dass** dabei Oberflächenstrukturen und Anfärbungen eingearbeitet werden.

17. Verfahren nach einem der Ansprüche 11 bis 16, **dadurch gekennzeichnet, dass** die erhaltenen Polymerfilme zur Verhinderung des Zusammenklebens mit einem Trennmittel, wie pflanzlicher Stärke oder Talkpuder, bestäubt werden.
